# EUROPEAN PATENT SPECIFICATION

(11) **EP 2 544 767 B1**
(45) Date of publication and mention of the grant of the patent: **19.12.2018**
(21) Application number: 11753716.7
(22) Date of filing: 09.03.2011
(51) Int. Cl.: A61N 7/00, A61N 7/02

(54) **ULTRASONC THERAPY APPLICATOR**
ULTRASCHALLTHERAPIEAPPLIKATOR
APPLICATEUR DE THÉRAPIE ULTRASONORE

(30) Priority: 09.03.2010 US 311847 P
(43) Date of publication of application: 16.01.2013
(73) Proprietor: Profound Medical Inc., Mississauga, ON L4W 5K5 (CA); Chopra, Rajiv, Toronto, Ontario M4N 3N1 (CA); Bronskill, Michael, Toronto, Ontario M4N 3N1 (CA); Donaldson, Sean, Toronto, Ontario M4N 3N1 (CA); Mahon, Cameron, Toronto, Ontario M4N 3N1 (CA)
(72) Inventor: CHOPRA, Rajiv, Ontario M4N 3N1 (CA); BRONSKILL, Michael, Ontario M4N 3N1 (CA); DONALDSON, Sean, Ontario M4N 3N1 (CA); MAHON, Cameron, Ontario M4N 3N1 (CA)
(74) Representative: DTS Patent- und Rechtsanwälte Schnekenbühl und Partner mbB
(86) International application number: PCT/US2011/000436
(87) International publication number: WO 2011/112250

(56) References cited:
- US-A- 5 593 415
- US-A- 5 593 415
- US-A- 6 077 257
- US-A1- 2002 087 156
- US-A1- 2006 206 105
- US-A1- 2006 206 105

## Description

### TECHNICAL FIELD

The present application relates to ultrasound therapy systems, and particularly to the construction and operation of an array of ultrasound sources for use in such systems.

### BACKGROUND

Ultrasonic transducers have been employed in ultrasound therapy systems to achieve therapeutic heating of diseased and other tissues. Phased ultrasound arrays of transducers operating to form a beam of ultrasonic energy cause a conversion of sound to thermal energy in the affected tissue areas or treatment volumes, and a subsequent beneficial rise in the temperature in the treatment volumes. With proper monitoring of the heating effect, ultrasound therapy systems can be used to treat harmful cells and to controllably destroy cancerous tumors.

As known to those skilled in the art, ultrasonic transducers are constructed and operated to take electrical power and produce ultrasound energy waves from a surface of a transducer element in a process generally referred to as transduction. The nature and extent of the transduction depends on the material used to construct the transducers, transducer geometry, and the electrical input to the transducers. A common material used in construction of ultrasound transducers is piezo-electric transducer crystal material (lead zirconate titanate, PZT) which comes in several forms.

Various designs for ultrasonic array systems have been used in the present field of art. The present disclosure will not provide a detailed exposition of the prior arrays. Ultrasound array design can be challenging, and improvements to such designs would improve the effectiveness, safety and cost to manufacture of such arrays. US 2006/206105 A1 describes an apparatus for thermal therapy in a patient having an elongated cylindrical portion having a first end thereof sized and configured for insertion into a male urethra, an array of ultrasonic sources disposed in the elongated cylindrical portion and arranged along an axis of the elongated cylindrical portion proximal to the first end of the elongated cylindrical portion. The ultrasonic sources are electrically driven to provide thermal therapy. A printed circuit board is disposed in the elongated cylindrical portion and includes a plurality of printed circuit lines respectively coupled to a plurality of ultrasonic sources of the array. The printed circuit lines provide power and control signals to the ultrasonic sources and drive the sources to deliver acoustic emissions of respective frequency and power depending on the respective power and control signals. The plurality of printed circuit lines is coupled to the plurality of ultrasonic sources of the array so as to create a separation between back sides of the ultrasonic sources and the circuit board so as to cause an outward radiation of ultrasonic energy from an outward face of the ultrasonic sources. A rotational mechanical coupling is placed proximal to the second end of the elongated cylindrical portion designed and is arranged to permit mechanical rotation of the elongated cylindrical portion and a fluid conduit runs through the rotational mechanical coupling permitting a fluid to circulate into and then out of the device by flowing from the second end towards the first end of the elongated cylindrical portion and back again.

### SUMMARY

The problems described above are obviated by a device for thermal therapy in a patient according to independent claim 1. Preferred embodiments of the invention are illustrated by the dependent claims. Embodiments hereof are directed to systems for providing an image-guided thermal therapy system including an ultrasonic array of transducers. In some respects, the present disclosure provides improved ultrasonic array designs to achieve better thermal therapy in such situations as trans-urethral prostate cancer therapy.

Aspects of the present disclosure provide a system with a computer controlled or microprocessor controlled RF driving unit that electrically drives piezo electric ultrasound transducer elements in an ultrasound therapy system as well as ways for coupling the arrays to a power source which can controllably power the elements of the arrays.

Some embodiments are directed to apparatus for thermal therapy in a patient, comprising an elongated cylindrical portion having a first end thereof sized and configured for insertion into a male urethra; an array of ultrasonic sources disposed within said elongated cylindrical portion and substantially arranged along an axis of said elongated cylindrical portion proximal to said first end of the elongated cylindrical portion, the ultrasonic sources being electrically driven to provide thermal therapy in said patient; a transition portion directly connected to a second end of said elongated cylindrical portion, said transition portion having a flared or bulbous shape that limits a depth of said insertion into said male urethra;an elongated printed circuit board disposed in said elongated cylindrical portion and extending from said first end to a second end of said elongated cylindrical portion, said circuit board including a plurality of printed circuit lines respectively coupled to a plurality of said ultrasonic sources of said array, said printed circuit lines providing power and control signals to said respective plurality of ultrasonic sources and driving said sources to deliver acoustic emissions of respective frequency and power depending on the respective power and control signals, said plurality of printed circuit lines on said circuit board being coupled to said plurality of ultrasonic sources of said array by way of respective epoxy points or pads of finite thickness so as to cause a gas-filled separation between back sides of said ultrasonic sources and said circuit board so as to cause an outward radiation of ultrasonic energy from an outward face of said ultrasonic sources; a rotational mechanical coupling proximal to a second end of said elongated cylindrical portion, said rotational mechanical coupling designed and arranged to permit mechanical rotation of said elongated cylindrical portion about said axis thereof; and at least one fluid conduit running through said rotational mechanical coupling permitting a fluid to circulate into and then out of said apparatus by flowing from said second end towards said first end of the elongated cylindrical portion and back again.

Other embodiments, not according to the present invention, are directed to method for thermal therapy in a patient, comprising providing a thermal therapy apparatus having an elongated portion thereof and preparing said apparatus for insertion into a urethra of said patient; inserting said elongated portion into said patient's urethra by translating at least a portion of said apparatus along an axis of said apparatus; delivering a plurality of electrical power and control signals to a corresponding plurality of ultrasonic elements disposed within said elongated portion, said electrical power and control signals providing at least frequency and power control driving signals to their respective ultrasonic elements; once the insertion step above has been accomplished satisfactorily, programmably rotating said apparatus about an axis of rotation, which axis of rotation is substantially the same as or parallel to said axis of said apparatus and its elongated portion; delivering sufficient ultrasonic energy from said plurality of ultrasonic sources into a diseased volume of said patient to effect a clinically-significant change in said diseased volume; and monitoring a result of said step of delivering said ultrasonic energy, including monitoring a temperature of said diseased volume.

### BRIEF DESCRIPTION OF THE DRAWINGS

For a fuller understanding of the nature and advantages of the present invention, reference is be made to the following detailed description of preferred embodiments and in connection with the accompanying drawings, in which:
Fig. 1 illustrates an exemplary system for providing image-guided ultrasound therapy to a patient;
Fig. 2 illustrates an exemplary design of an elongated ultrasound thermal therapy applicator;
Fig. 3 illustrates an exemplary ultrasonic array for use in an ultrasound therapy system;
Fig. 4 illustrates two views of an elongated ultrasonic thermal therapy applicator according to exemplary embodiments hereof;
Fig. 5 illustrates an exemplary sequence for a method used in ultrasound thermal therapy;
Fig. 6 illustrates a view of the circuit support member such as a PCB support member; and
Fig. 7 illustrates exemplary designs of two ends of an ultrasound treatment applicator, one for insertion into a treatment volume of a patient's body and the other for coupling to mechanical and electrical components of the treatment system.

### DETAILED DESCRIPTION

As discussed above, improved ultrasound thermal therapy applicators can improve treatment of diseases such as tumors, and for example as used in trans-urethral treatment of prostate cancers in male patients.

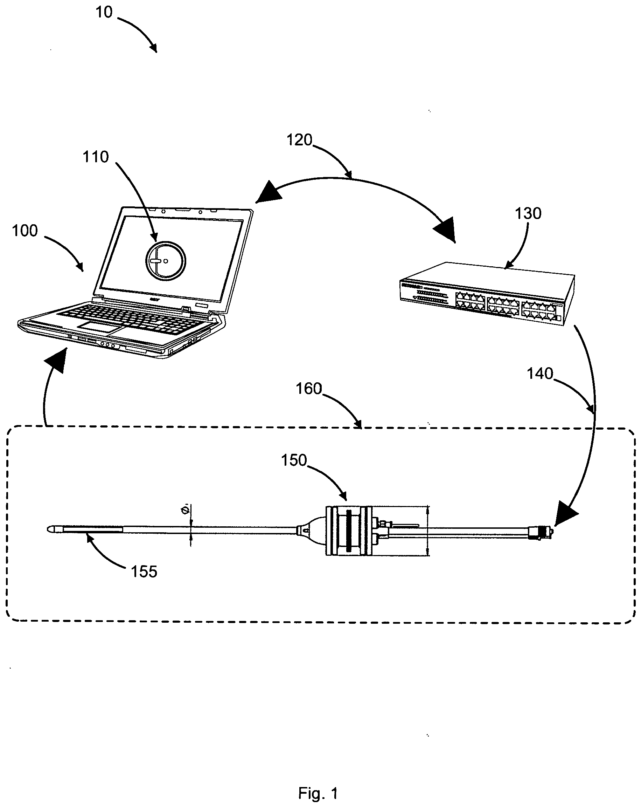

Fig. 1 illustrates an exemplary system 10 for providing image-guided ultrasound therapy to a patient. The simplified illustration shows a master computer 100, such as a portable PC, workstation, or other processing device having a processor, memory, and coupled to some input/output apparatus. Master computer 100 may include a display and may support a user interface 110 to facilitate control of and observation of the thermal therapy treatment process.

Master computer 100 is adapted for coupling to other systems and components through a computer interface connector 120. Connection 120 carries data and information to and from master computer 100 and may comprise standard or special-purpose electrical wiring connection cables, such as serial connection cables or the like. Also, connection 120 may be achieved wirelessly as known to those skilled in the art of wireless communication, and may further be achieved by way of multiple connections, over a network, or by another suitable method.

In some embodiments, master computer 100 is coupled through connection 120 to a power control unit 130. Power control unit 130 may be implemented as a stand-alone hardware apparatus but may be implemented as a part of master computer 100, e.g., by being built onto a special card in a computer or server system that accommodates such hardware components.

Power control unit 130 may specifically include at least a processor adapted for processing machine or program instructions, which may be provided to the processor from another component of system 10 and may be stored on a memory device in power control unit 130. Circuitry including analog and/ or digital circuitry may be operated within power control unit 130 so as to determine an output power to one or more ultrasound therapy transducer elements in an ultrasound therapy apparatus 150.

In some embodiments, power control unit 130 may deliver controlled electrical driving signals to a plurality of ultrasound transducer elements (e.g., PZT array elements) in ultrasound therapy apparatus 150. The driving signals may be controlled to deliver a programmed amount of power to each element or to groups of elements of therapy apparatus 150. The driving signals may also be controlled so as to provide a determined driving voltage, current, amplitude, waveform, or frequency to said ultrasonic transducers of therapy apparatus 150. Such electrical driving signals are carried from power control unit 130 to the ultrasound therapy apparatus 150 over suitable wires, cables, or buses 140. Appropriate plug interfaces or connectors may be included so as to mate the various ends of the connectors or buses to and from their associated components.

In operation, ultrasound therapy apparatus 150 includes a portion 155 that is inserted into a portion of a patient's body to deliver a suitable dose of ultrasound energy to tissue in a diseased region of the patient's body.

The patient and the ultrasound therapy apparatus 150 are generally disposed in an imaging volume 160 such as a magnetic resonance imaging (MRI) apparatus, which can provide real-time images of the relevant parts of the patient, e.g., the treatment volume to master computer 100 or display and user interface 110. In some embodiments, real-time monitoring of the thermal therapy is performed so that a clinical operator can monitor the progress of the therapy within the treatment volume or diseased tissue. Manual or automated changes can be made to the power signals from power control unit 130 based on input from the results and progress of the treatment.

The feedback and coupling of the treatment system components to the control components in system 10 can be used to ensure that an optimum radio frequency (RF) power signal is provided to each element of an ultrasound array 155 used in treatment of diseased tissues. Some examples include treatment of prostate cancer tumors in male patients using MRI guided ultrasound therapy applications.

RF power control unit 130 may include separate circuit cards having individual processors, amplifiers, filters and other components to achieve the desired driving power output to the elements of ultrasound array 155 of ultrasound treatment apparatus 150. Alternatively, a single processor may be employed to control the behavior of the various power channels to each array element.

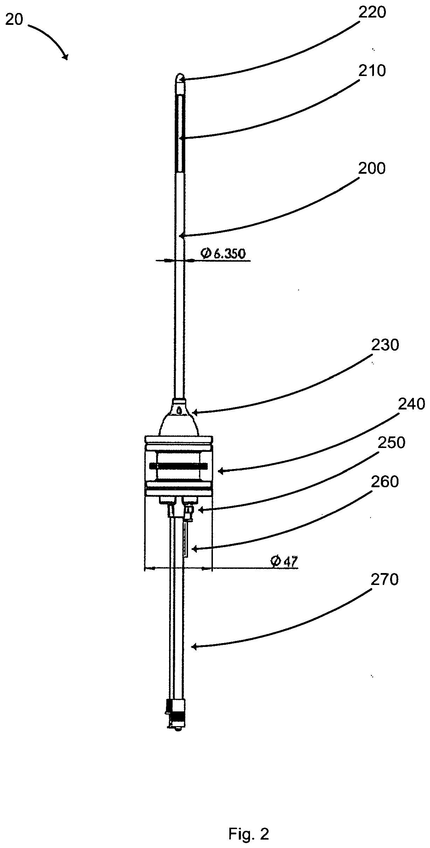

Fig. 2 illustrates an exemplary ultrasound therapy applicator design. Applicator 20 includes an elongated shaft portion 200, which can be inserted into a body cavity proximal to a diseased tissue region of a patient. In some instances, elongated shaft portion 200 (or a portion thereof) may be inserted into the urethra of a male patient to treat diseased tissue such as cancerous tissue of the male prostate. The insertion of applicator 20 into the patient is done by pressing the applicator 20 into an appropriate channel, optionally using image guidance such as MRI or X-ray guidance to monitor the movement of applicator 20 within the patient.

The applicator 20, or typically the elongated shaft portion 200, are inserted into the patient until the transducer array 210 reaches an area proximal to the diseased tissue volume or target volume for the thermal therapy. In this way, when power is provided to the transducer array 210 it will cause a controlled heating of the diseased tissue volume to treat the disease condition or affect some other desired outcome. Tip 220, as will be discussed below, may be constructed of blunt smooth material such as a polymer or metal material to assist in easy reduced friction insertion and movement of applicator 20 into the patient. In some embodiments, this design minimizes frictional stress on the interior walls of the patient's urethra.

A transition portion 230 of applicator 20 is flared or bulbous in shape and provides a safety zone that prevents unwanted portions of apparatus 20 from entering into the patient's body.

Flanged portions of transition portion 230 allow for easier manipulation of applicator 20 and mechanical control of the same as will be described below in further detail. The portion 230 can act as a handle for holding the applicator and may be constructed of an optically transparent material such as clear plastic. This can allow viewing of the interior of the apparatus in some situations to determine if any gas (air) bubbles have been trapped in the fluid circuit portion of the apparatus. The gas can then be vacated to minimize or avoid interference in the transmission of ultrasound energy from the transducer system or interference with the cooling fluid flow within the body of the system. The flanges can also provide a mechanical means for holding applicator 20 in place within a bearing system or rotation and translation driver used to move and rotate applicator 20 during operation.

A geared element 240 provides a mechanically-compatible actuation means for rotating applicator 20 within the patient's body so that array 210 is properly and controllably rotated about the long axis of shaft 200 to treat a volume of tissue up to a complete 360 degree rotation volume surrounding the axis of shaft 200 if desired. In some embodiments, a motor is adapted for driving the gear 240 of applicator 20 to provide such rotation of the applicator within the patient about the long axis of the applicator.

Mechanical interfaces 250 allow coupling of fluid intake and outtake connections to applicator 20 so that temperature control fluid can be passed into and out of the applicator 20. For example, in situations where cooling of the applicator itself or surrounding tissue in needed, the fluid can be applied to these interfaces optionally using standard fluid hook-up connectors and tubing 270. Also, electrical wiring 260 or micro-buses can be passed through interfaces 250 to provide electrical driving power to the elements of transducer array 210 and to receive sensor signals or other signals and data from the components of applicator 20. Again, standard electrical connectors may be used to interface outside power and control systems with the internal electrical elements of applicator 20.

In operation, applicator 20 may be placed with tip 220 proximal to an aperture in the patient's body and with the long axis of shaft 200 substantially aligned with a cavity or channel (e.g., the urethra) of the patient for insertion therein. The applicator 20 is then automatically or manually or by a combination of the two inserted into the patient's body, beginning with tip 220 end of shaft 200. When the applicator 20 is sufficiently inserted into the patient's body (e.g., using image guided translation motor stages) the translation of applicator 20 is secured. Then, a computer-controlled thermal therapy procedure is undertaken, with applicator 20 being rotated about its long axis within the patient's body so that transducer array 210 provides a therapeutic energy field such as an ultrasonic field of known strength and nature to treat the diseased tissue proximal to array 210. When the thermal therapy is completed, power to ultrasound array 210 is secured and applicator 20 is retracted from the patient substantially along the long axis of the applicator, in substantially the reverse direction as it was inserted.

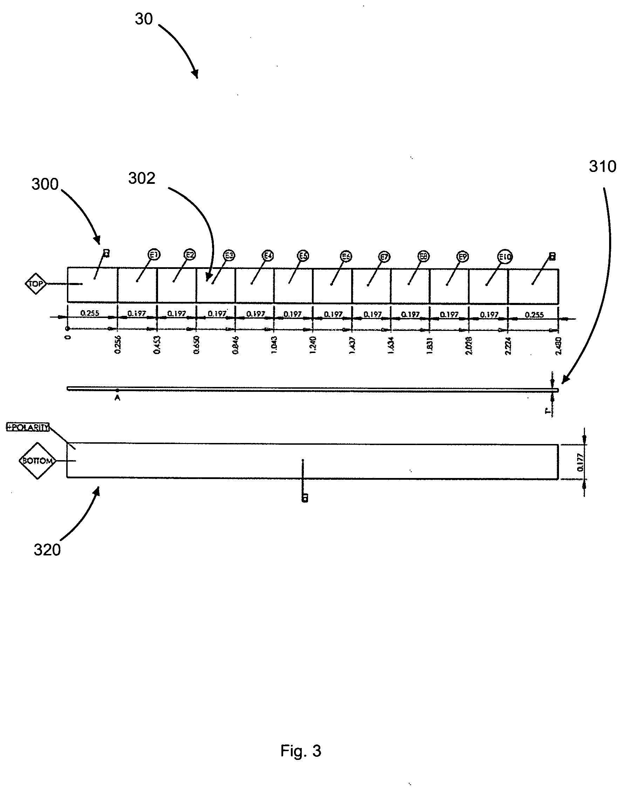

Fig. 3 illustrates an exemplary design for the transducer array (such as array 210 of Fig. 2). An ultrasonic array 30 is shown as it appears from the "top" face thereof at view 300. Note that in the present illustration the "top" face is the face of the array normally facing into the center of the applicator shaft and away from the patient's body. The same array is shown from the side in view 310. The opposing or "bottom" view of the array is shown in view 320, and is the face of the array which is outwardly directed at the patient's treatment volume and away from the applicator. It is seen that in this exemplary embodiment the ultrasonic array is constructed from a substantially flat or relatively planar material. This may be a PZT-based material as is generally known to those skilled in the art. In some embodiments the material may comprise K320 from Piezo Technologies of Indianapolis, IN USA. Alternately, it may be made of PZ52 of similar material from Ferroperm Piezoceramics of Kvistgaard, Denmark. The array and its elements may be designed and arranged to have a pre-determined optimal resonance frequency, for example 4 MHz, or other central frequency for best penetration and power delivery to the diseased volume of tissue, in some embodiments, along with a third harmonic at 13 MHz as well.

According to the present embodiment, the front face of transducer array 30 is cut into a plurality of individual array elements, e.g., 302. The individual elements 302 may or may not all be of the same shape and size. The dimensions given in the figure are merely illustrative. In certain embodiments, the elements 302 are substantially rectangular or square in shape and provide an ultrasonic energy field proximal to the face of elements 302 as dictated by the design, material and driving signals for the elements 302. The elements 302 of array 30 may be driven in a programmed way as discussed in other applications by the present inventors and assignee to create an overall ultrasonic therapeutic energy field within a controlled volume of tissue in a patient. The array 30 mounted to the rest of the therapy applicator may be rotated about the long axis of array 30 so as to provide treatment to a volume around array 30 as needed.

Both the front face 300 and the back face 320 of array 30 are silvered to permit delivery of driving power signals to and grounding of the elements of array 30. The ends and edges (shown in 310) of array 30 may be left unsilvered. In this way some or all of elements 302 may be powered by an appropriate power source.

In some embodiments, one or both elements at the ends of array 30 may be "dummy" elements that are not substantially driven or used for the actual thermal therapy in operation of the device.

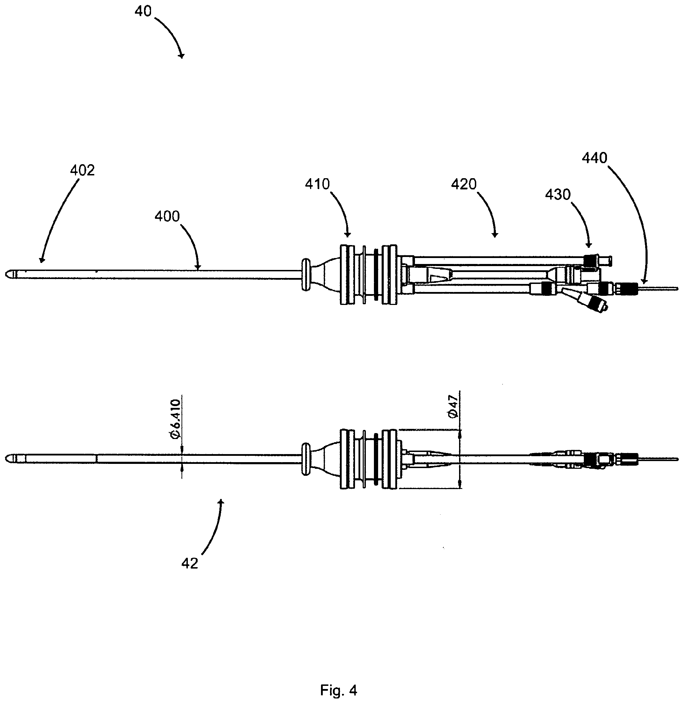

Fig. 4 illustrates two views 40 and 42 of an illustrative ultrasound therapy applicator device, showing the exemplary arrangement of the connectors and transitional mechanical elements thereof with respect to the elongated shaft and transducer array portions as described above. An elongated portion 400 is designed for insertion into a male urethra, optionally by applying an acoustically-compatible lubricant or disinfecting liquid or gel to an exterior of elongated portion 400. Ultrasound elements are arranged within and running along a portion near the tip 402 of elongated applicator 400. This is the part of the apparatus which is inserted into the patient's body until it is substantially situated within a volume of diseased tissue (e.g., the prostate) and from which the ultrasonic thermal energy is emitted into the diseased tissue.

The elongated portion is supported by and secured to one or more flanged elements of the applicator body, which in a preferred embodiment act as bearings 410 or gear elements to assist in rotating the applicator about its long axis once the applicator's tip is at the desired depth within the patient. In some aspects, a motorized driver as described elsewhere by the present applicant is used to mechanically rotate and/or translate the apparatus.

For example, in an embodiment, not according to the present invention, the applicator 40, 42 is inserted into a patient who is lying on and secured to a bed, table, or platform. Once inserted to the proper position in the patient so that the ultrasonic array in portion 402 of the applicator is proximal to the diseased tissue, a rotational stepper motor or other piezo-electric driver is used to mechanically turn the apparatus and hence the ultrasonic array of the apparatus about its axis so as to sonicate the diseased tissue (prostate) to the desired degree using computer-controlled power, frequency or other electrical driving signals delivered to the elements of the array at 402.

As discussed elsewhere in this disclosure, electrical and mechanical (e.g., fluid) connections are made from portions of the applicator outside the patient's body to portions of the applicator inserted into the patient's body. Preferably, such mechanical and electrical connections employ physically compact components to reduce the discomfort felt by the patient and to reduce the chances of strain on the patient's healthy organs (e.g., urethra). Accordingly, in an embodiment, fluid conduits 420 into and out of the patient are provided with appropriate transitional or coupling ends and deliver electrical or fluid content to and from the elongated portion 400 and proximal to tip end 402 of the apparatus. Further coupling using fluid couplings 430 and electrical couplings 440 are provided, and these couplings are connected to corresponding parts of the fluid circuit pumping fluid into the applicator and out of the same and electrical circuits delivering power and control capability to the system, respectively.

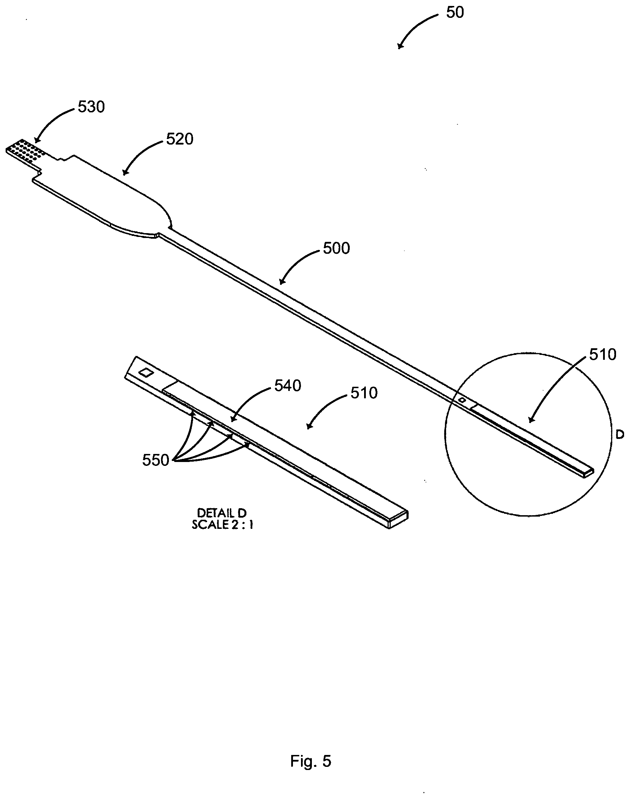

Fig. 5 illustrates an exemplary internal view of the transducer support and assembly member 50, on which the ultrasonic array is supported and on which the circuitry for driving the elements of the array are mounted. In the present embodiment, an elongated substantially flattened and paddle-like shaped support member 50 has a long shaft section 500. Ultrasonic array 510 is disposed near a first end of said shaft section, and array 510 may be attached, fixed, mounted or adhered to said long section 500 by any means convenient or effective for a particular application and geometry.

A wider section 520 extends from a second end of elongated section 500 and is placed within a transitional portion of the therapy applicator and is generally not inserted into the patient's body. An electrical connection 530 is provided for connecting to the outside electrical power drive and control system.

In some embodiments the support and assembly member 50 is made of or on or includes a printed circuit board (PCB) material. On the PCB, thin electrical connections are printed and run from electrical connector 530 up the shaft 500 to power the elements of transducer array 510.

A detail "D" of the array 510 end of the system is shown below in the same drawing. The common ground "bottom" face 540 of the transducer array is shown, as are several connection points 550 to the "top" face of the individual transducer elements on the opposing face of array 510. The individual wiring can be accomplished by placement of the array onto the PCB support member and soldering of connections between the PCB circuitry and the individual array elements so as to allow individual power and control of the same.

An inter-metallic bond or epoxy connection points can be used to couple the transducer elements to the PCB lines. The connection points form "pads" of a finite thickness. These pads cause the surface of the PCB and the surface of the transducers to be separated (e.g., by a thickness of about 0.003 inch). 3-oz copper pad connection points will provide approximately a 0.0034 inch air gap. The separation is air-filled or gas-filled so as to provide an "air backing" to the transducer array 510 so that the array directs its energy outwardly from the "bottom" face thereof, facing the patient, as opposed to radiating its energy through the top face or another direction. This spacing of the array and the support structure 500 is a design feature that eliminates the need for using a spacer to provide the air-backing in some embodiments. It is noted that the present exemplary dimensions and arrangements are given for the sake of illustration, and are not limiting, so that one of skill in the art would appreciate other forms and sizes and arrangements accomplishing substantially same or similar ends in similar ways.

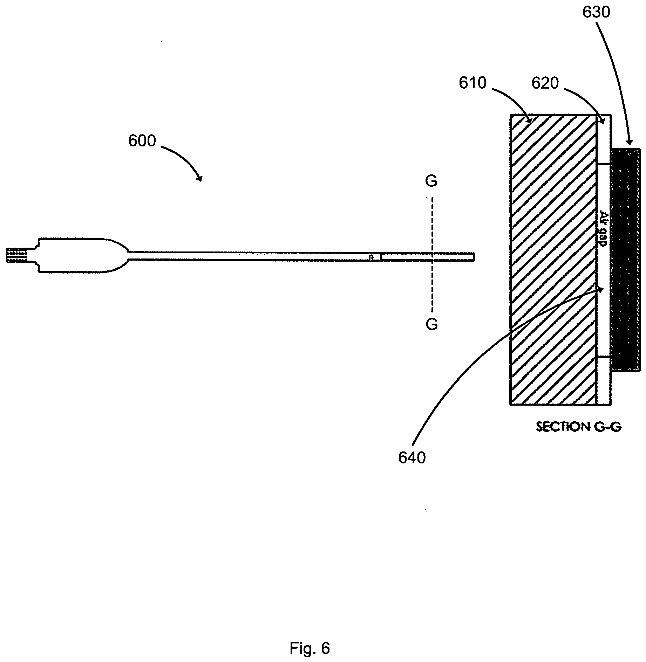

Fig. 6 illustrates a view of the circuit support member 600 such as the PCB support member described above. A section G-G is shown to the right to illustrate an exemplary arrangement of the silvered transducer element 630, which is coupled to the PCB stalk material 610 by copper or other conducting pads 620. The pads 620 are of a thickness as described above to provide a suitable air gap 640 so that the transducers 630 are properly air-backed for transmitting ultrasonic energy from the bottom face of the elements 630 (to the right in Fig. 6) towards the diseased tissue of the patient.

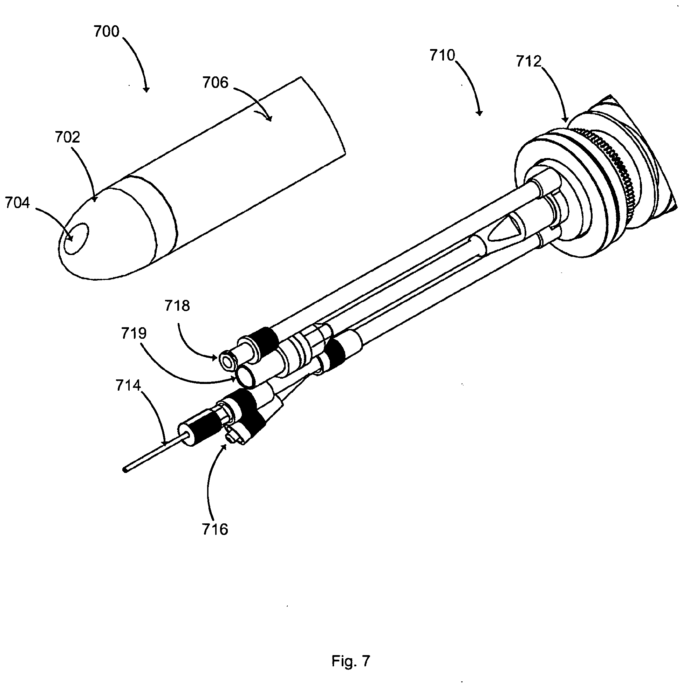

Fig. 7 illustrates exemplary designs of two ends of an ultrasound treatment applicator.

At one end 700 of the applicator, as discussed earlier, is a tip portion 702 coupled to the inserted end of the elongated shaft member 706 of the applicator. In some embodiments, a fiber optic or other temperature sensor is placed at or near the tip of the applicator for sensing the temperature in or near the tip of the applicator.

In some embodiments, a hole 704 or small orifice is disposed at or near the leading end of tip 702. The hole allows for drainage of fluid, e.g. urine that may collect in the patient near the tip end of the applicator. This can reduce the swelling or pressure in the patient near the treatment zone during a thermal therapy procedure. The fluid drained from the patient through hole 704 may be carried in a tube or channel down the length of the applicator apparatus to the opposite end of the applicator and outside the patient at exterior end 710 of the applicator.

End 710 of the thermal therapy applicator includes a catheter 714 in fluid communication with the hole 704 in tip 702. This catheter delivers fluid (e.g., urine) drained from the patient's body to a suitable retainer or receiving volume. The drained fluid can be monitored for blood, drugs, temperature, or other attributes. A valve or shut-off apparatus may be included in-line with catheter 714 to control the flow of fluid in or out of the catheter. In some embodiments fluid may be delivered in to the patient's body, including drug delivery to the patient near the tip 702 of the applicator.

The present invention should not be considered limited to the particular embodiments described above. Various modifications, equivalent processes, as well as numerous structures to which the present invention may be applicable, will be readily apparent to those skilled in the art to which the present invention is directed upon review of the present disclosure.

## Claims

1. An apparatus for thermal therapy in a patient, comprising:
an elongated cylindrical portion (200) having a first end (220) thereof sized and configured for insertion into a male urethra;
an array of ultrasonic sources (510) disposed within said elongated cylindrical portion and substantially arranged along an axis of said elongated cylindrical portion proximal to said first end of the elongated cylindrical portion, the ultrasonic sources being electrically driven to provide thermal therapy in said patient;
a transition portion (230) directly connected to a second end of said elongated cylindrical portion, said transition portion having a flared or bulbous shape that limits a depth of said insertion into said male urethra;
an elongated printed circuit board (50) disposed in said elongated cylindrical portion and extending from said first end to a second end of said elongated cylindrical portion, said circuit board including a plurality of printed circuit lines respectively coupled to a plurality of said ultrasonic sources of said array, said printed circuit lines providing power and control signals to said respective plurality of ultrasonic sources and driving said sources to deliver acoustic emissions of respective frequency and power depending on the respective power and control signals, said plurality of printed circuit lines on said circuit board_being coupled to said plurality of ultrasonic sources of said array by way of respective epoxy points or pads of finite thickness so as to cause a gas-filled separation between back sides of said ultrasonic sources and said circuit board so as to cause an outward radiation of ultrasonic energy from an outward face of said ultrasonic sources;
a rotational mechanical coupling (240) proximal to a second end of said elongated cylindrical portion, said rotational mechanical coupling designed and arranged to permit mechanical rotation of said elongated cylindrical portion about said axis thereof; and
at least one fluid conduit (270) running through said rotational mechanical coupling permitting a fluid to circulate into and then out of said apparatus by flowing from said second end towards said first end of the elongated cylindrical portion and back again.

2. The apparatus of claim 1, further comprising a geared wheel proximal to said rotational mechanical coupling, designed and arranged to engage with a corresponding mechanical drive element so as to cause rotation of said apparatus about said axis and to therefore allow thermal therapy treatment of a volume of diseased tissue substantially surrounding said first end of said elongated cylindrical portion.

3. The apparatus of claim 1, further comprising an orifice substantially proximal to an extremity of said elongated cylindrical portion, the orifice arranged to draw a body fluid from an interior of said patient, through said elongated cylindrical portion and out of the apparatus.

4. The apparatus of claim 1, said array of ultrasonic sources being disposed on an elongated support plate having a first elongated section shaped and sized to fit within an interior cavity in said elongated cylindrical portion proximal to said first end and having a wider section having a flattened and paddle-like shape disposed within said transition portion, said support plate comprising said circuit board.

5. The apparatus of claim 4, said plurality of printed circuit lines fixed to said support plate, the printed circuit lines electrically connected at one end thereof to said ultrasonic sources and electrically connected at another end thereof to a source of said power and control signals.

6. The apparatus of claim 1, further comprising mechanical fluid couplings at an end thereof and distal from said first end of the elongated cylindrical portion, the mechanical fluid couplings respectively connectable to a fluid supply line and to a fluid discharge line for circulating fluid into and out of said apparatus.

7. The apparatus of claim 1, said apparatus comprising substantially magnetic field compatible components allowing the apparatus to be used in a magnetic resonance imaging (MRI) treatment facility without substantially interfering with the operation of such magnetic imaging.

8. The apparatus of claim 7, said apparatus possessing a substantially radially symmetrical magnetic profile so that rotation of said apparatus during treatment within a magnetic imaging field would have little or no detrimental impact on magnetic imaging performed in conjunction with said treatment.

## Patentansprüche

1. Vorrichtung zur Wärmetherapie in einem Patienten, aufweisend:
einen länglichen zylindrischen Abschnitt (200) mit einem ersten Ende (220), das so bemessen und dazu ausgelegt ist, in eine Harnröhre eines Mannes eingeführt zu werden;
eine Anordnung aus Ultraschallquellen (510), die innerhalb des länglichen zylindrischen Abschnitts vorgesehen und im Wesentlichen entlang einer Achse des länglichen zylindrischen Abschnitts im Nahbereich des ersten Endes des länglichen zylindrischen Abschnitts angeordnet sind, wobei die Ultraschallquellen elektrisch angesteuert werden, um eine Wärmetherapie im Patienten bereitzustellen;
ein Übergangsabschnitt (230), der direkt mit einem zweiten Ende des länglichen zylindrischen Abschnitts verbunden ist, wobei der Übergangsabschnitt eine aufgeweitete oder bauchige Form hat, die eine Einführtiefe in die Harnröhre eines Mannes begrenzt;
eine längliche Leiterplatte (50), die in dem länglichen zylindrischen Abschnitt vorgesehen ist und sich vom ersten Ende zu einem zweiten Ende des länglichen zylindrischen Abschnitts erstreckt, wobei die Leiterplatte eine Vielzahl von gedruckten Schaltungsleitungen aufweist, die jeweils an eine aus der Vielzahl der Ultraschallquellen der Anordnung angeschlossen sind, wobei die gedruckten Schaltungsleitungen den jeweiligen mehreren Ultraschallquellen Energie- und Steuersignale bereitstellen und diese ansteuern, um akustische Abstrahlungen mit jeweiliger Frequenz und Energie je nach den entsprechenden Energie- und Steuersignalen abzugeben, wobei die Vielzahl der gedruckten Schaltungsleitungen auf der Leiterplatte an die mehreren Ultraschallquellen der Anordnung mittels jeweiliger Epoxidpunkte oder -stellen endlicher Dicke angeschlossen sind, um eine gasbefüllte Trennung zwischen den Rückseiten der Ultraschallquellen und der Leiterplatte zu bewirken, um eine nach außen gerichtete Abstrahlung von Ultraschallenergie von einer nach außen gerichteten Fläche der Ultraschallquellen herbeizuführen;
eine mechanische Drehkupplung (240) nahe einem zweiten Ende des länglichen zylindrischen Abschnitts, wobei die mechanische Drehkupplung dazu ausgelegt und eingerichtet ist, eine mechanische Drehung des länglichen zylindrischen Abschnitts um dessen Achse zu ermöglichen; und
mindestens eine Fluidleitung (270), die durch die mechanische Drehkupplung verläuft und ermöglicht, dass ein Fluid umlaufend in die Vorrichtung hinein und aus dieser heraus fließen kann, indem es vom zweiten Ende zum ersten Ende des länglichen zylindrischen Abschnitts und wieder zurück strömt.

2. Vorrichtung nach Anspruch 1, darüber hinaus ein Zahnrad im Nahbereich der mechanischen Drehkupplung aufweisend, das so ausgelegt und eingerichtet ist, dass es mit einem entsprechenden mechanischen Antriebselement in Eingriff steht, um eine Drehung der Vorrichtung um die Achse zu bewirken und dadurch eine Wärmetherapiebehandlung eines Volumens an erkranktem Gewebe zu ermöglichen, das im Wesentlichen das erste Ende des länglichen zylindrischen Abschnitts umgibt.

3. Vorrichtung nach Anspruch 1, darüber hinaus eine Mündung aufweisend, die sich im Wesentlichen im Nahbereich eines äußersten Endes des länglichen zylindrischen Abschnitts befindet, wobei die Mündung dazu eingerichtet ist, eine Körperflüssigkeit aus dem Inneren des Patienten durch den länglichen zylindrischen Abschnitt hindurch und aus der Vorrichtung heraus abzuziehen.

4. Vorrichtung nach Anspruch 1, wobei die Anordnung der Ultraschallquellen auf einer länglichen Trägerplatte vorgesehen ist, die einen ersten länglichen Teilbereich, der so gestaltet und bemessen ist, dass er sich in einen Innenhohlraum im länglichen zylindrischen Abschnitt nahe dem ersten Ende einpasst, und einen breiteren Teilbereich aufweist, der eine abgeflachte und paddelartige Form hat, die innerhalb des Übergangsabschnitts vorgesehen ist; wobei die Trägerplatte die Leiterplatte aufweist.

5. Vorrichtung nach Anspruch 4, wobei die Vielzahl der gedruckten Schaltungsleitungen an der Trägerplatte befestigt sind, und die gedruckten Schaltungsleitungen an einem Ende jeweils an die Ultraschallquellen und am anderen Ende jeweils an eine Quelle der Energie- und Steuersignale elektrisch angeschlossen sind.

6. Vorrichtung nach Anspruch 1, darüber hinaus mechanische Fluidkupplungen an einem Ende von dieser und distal vom ersten Ende des länglichen zylindrischen Abschnitts aufweisend, wobei die mechanischen Fluidkupplungen an eine Fluidzufuhrleitung bzw. eine Fluidabfuhrleitung für eine Umlaufströmung des Fluids in die Vorrichtung hinein und aus dieser heraus anschließbar sind.

7. Vorrichtung nach Anspruch 1, wobei die Vorrichtung im Wesentlichen magnetfeldkompatible Komponenten aufweist, die es ermöglichen, dass die Vorrichtung in einer Magnetresonanz-Bildgebungs-(MRI)-Behandlungsanlage eingesetzt werden kann, ohne dabei den Ablauf einer derartigen Magnettomographie in nennenswertem Maße zu stören.

8. Vorrichtung nach Anspruch 7, wobei die Vorrichtung ein im Wesentlichen radialsymmetrisches Magnetprofil hat, so dass eine Drehung der Vorrichtung während der Behandlung innerhalb eines Magnettomographiefeldes eine geringe oder gar keine negative Auswirkung auf die in Verbindung mit der Behandlung durchgeführte Magnettomographie hat.

## Revendications

1. Appareil de traitement thermique au sein d'un patient, comprenant :
une partie cylindrique allongée (200) ayant une première extrémité (220) correspondante dimensionnée et configurée pour l'insertion dans un urètre mâle ;
une batterie de sources ultrasoniques (510) disposées à l'intérieur de ladite partie cylindrique allongée et sensiblement agencée le long d'un axe de ladite partie cylindrique allongée de façon proximale par rapport à ladite première extrémité de la partie cylindrique allongée, les sources ultrasoniques étant entraînées électriquement de façon à apporter un traitement thermique au sein dudit patient ;
une partie de transition (230) reliée directement à une seconde extrémité de ladite partie cylindrique allongée, ladite partie de transition ayant une forme évasée ou bulbeuse limitant une profondeur de ladite insertion dans ledit urètre mâle ;
une carte de circuit imprimé (50) allongée disposée dans ladite partie cylindrique allongée et s'étendant hors de ladite première extrémité en direction d'une seconde extrémité de ladite partie cylindrique allongée, ladite carte de circuit imprimé comprenant une pluralité de lignes de circuit imprimé respectivement couplées à une pluralité de sources ultrasoniques de ladite batterie, lesdites lignes de circuit imprimé apportant les signaux de courant et de commande à ladite pluralité respective de sources ultrasoniques et amenant lesdites sources à délivrer des émissions acoustiques de fréquence et courant respectifs en fonction des signaux de courant et de commande respectifs, ladite pluralité de lignes de circuit imprimé sur ladite carte de circuit imprimé étant couplée à ladite pluralité de sources ultrasoniques de ladite batterie à l'aide de points ou coussins époxy respectifs d'épaisseur finie de façon à provoquer une séparation remplie de gaz entre les faces arrière desdites sources ultrasoniques et ladite carte de circuit imprimé de façon à provoquer un rayonnement d'énergie ultrasonique vers l'extérieur depuis une face extérieure desdites sources ultrasoniques ;
un couplage mécanique tournant (240) placé de façon proximale par rapport à une seconde extrémité de ladite partie cylindrique allongée, ledit couplage mécanique tournant étant conçu et agencé pour permettre la rotation mécanique de ladite partie cylindrique allongée autour dudit axe correspondant ; et
au moins un conduit de fluide (270) passant à travers ledit couplage mécanique tournant permettant à un fluide de circuler dans ledit appareil puis hors de celui-ci en circulant de ladite seconde extrémité en direction de ladite première extrémité de la partie cylindrique allongée et inversement.

2. Appareil selon la revendication 1, comprenant en outre une roue engrenée placée de façon proximale par rapport audit couplage mécanique tournant, ladite roue étant conçue et agencée pour s'engrener avec un élément d'entraînement mécanique correspondant de façon à provoquer la rotation dudit appareil autour dudit axe et ainsi permettre le traitement thérapeutique thermique d'un volume de tissu endommagé entourant sensiblement ladite première extrémité de ladite partie cylindrique allongée.

3. Appareil selon la revendication 1, comprenant en outre un orifice sensiblement placé de façon proximale par rapport à une extrémité de ladite partie cylindrique allongée, l'orifice étant agencé pour extraire un fluide corporel d'un intérieur dudit patient en passant à travers ladite partie cylindrique allongée jusqu'à la sortie de l'appareil.

4. Appareil selon la revendication 1, ladite batterie de sources ultrasoniques étant disposée sur une plaque de support allongée ayant une première section allongée formée et dimensionnée de façon à s'ajuster à l'intérieur d'une cavité intérieure dans ladite partie cylindrique allongée située de façon proximale par rapport ladite première extrémité et ayant une section plus large ayant une forme aplatie et de type rame disposée à l'intérieur de ladite partie de transition, ladite plaque de support comprenant ladite carte de circuit imprimé.

5. Appareil selon la revendication 4, ladite pluralité de lignes de circuit imprimé étant fixée à ladite plaque de support, les lignes de circuit imprimé étant connectées électriquement au niveau d'une extrémité correspondante auxdites sources ultrasoniques et étant connectées électriquement au niveau d'une autre extrémité correspondante à une source desdits signaux de courant et de commande.

6. Appareil selon la revendication 1, comprenant en outre des couplages fluides mécaniques au niveau d'une extrémité correspondante et placés de façon distale par rapport à ladite première extrémité de la partie cylindrique allongée, les couplages fluides mécaniques pouvant respectivement être connectés à une ligne d'alimentation en fluide et à une ligne de décharge de fluide pour faire circuler le fluide dans l'appareil et hors de celui-ci.

7. Appareil selon la revendication 1, ledit appareil comprenant un champ sensiblement magnétique compatible avec des composants permettant d'utiliser l'appareil dans un équipement de traitement par imagerie par résonance magnétique (IRM) sans interférer sensiblement avec le fonctionnement d'une telle imagerie magnétique.

8. Appareil selon la revendication 7, ledit appareil possédant un profil magnétique sensiblement symétrique dans le plan radial de sorte que la rotation dudit appareil pendant le traitement à l'intérieur d'un champ d'imagerie magnétique aurait un impact réduit non préjudiciable sur l'imagerie magnétique effectuée conjointement avec ledit traitement.
